# EUROPEAN PATENT APPLICATION

(11) **EP 2 243 485 A1**
(43) Date of publication of application: **27.10.2010**
(21) Application number: 09711707.1
(22) Date of filing: 20.02.2009
(51) Int. Cl.: A61K 31/555, A61P 31/16, D06M 13/503

(54) **ANTI-VIRAL AGENTS, ANTI-VIRAL FIBERS AND ANTI-VIRAL FIBER STRUCTURES**

(30) Priority: 20.02.2008 JP 2008039344; 04.07.2008 JP 2008176181
(71) Applicant: Daiwabo Holdings Co., Ltd., Chuo-ku Osaka-shi, Osaka 541-0056 (JP)
(72) Inventor: MATSUSHITA, Miki, Kako-gun Hyogo 675-0163 (JP); OTSUKI, Koichi, Kyoto-shi Kyoto 603-8555 (JP); TAKAKUWA, Hiroki, Kyoto-shi Kyoto 603-8555 (JP); TSUNEKUNI, Ryota, Kyoto-shi Kyoto 603-8555 (JP)
(74) Representative: von Kreisler Selting Werner
(86) International application number: PCT/JP2009/053084
(87) International publication number: WO 2009/104761

(57) **Abstract**

Provided are antiviral agents that are effective in inactivating viruses, and fiber products and fiber structures carrying these antiviral agents. The antiviral agent effectives against influenza and comprises a metal phthalocyanine derivative represented by formula I. Fiber carries these antiviral agents.

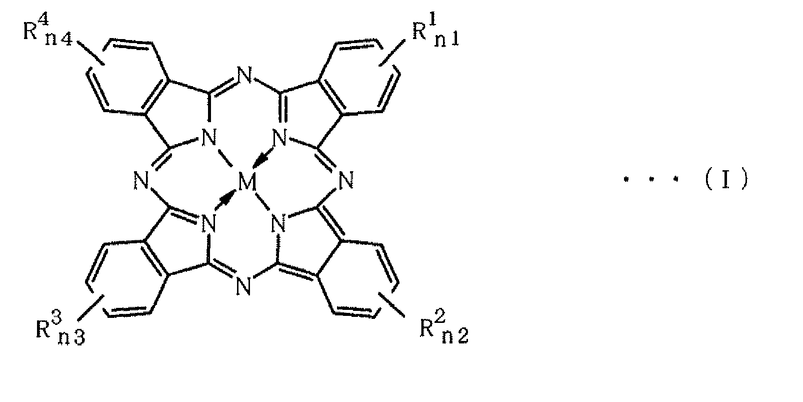

## Description

### Technical Field

The present invention relates to antiviral agents comprising a metal phthalocyanine derivative as a main component that is effective against viruses, and to fiber having an antiviral function.

### Background of the Invention

Recently, viral diseases such as SARS (Severe Acute Respiratory Syndrome) and avian influenza are rampant worldwide. As for influenza viruses, in particular, new types thereof have been found one after another, hence posing a threat to humans, Originally, the host range of a virus is limited so that a virus that infects mammals has only infected mammals and another virus that infects birds has only infected birds. However, the avian influenza viruses have a wide host range and can infect mammals as well as birds so that humans could be infected with this virus. Recently, H5N1 type influenza is rampant in Asia and Europe and accordingly it is feared that a new type of human influenza derived from the avian influenza could appear.

In addition, the avian influenza viruses are conveyed to remote areas by migratory birds so that it is difficult to prevent the viruses from entering into a country only by quarantine or suspension of, for example, food imports from a country where the avian influenza occurs.

An agent to inactivate influenza viruses is disclosed in Japanese Unexamined Patent Publication No. 2006-328039. This influenza virus inactivating agent is liquid comprising iodine and β-cyclodextrin.

In Japanese Unexamined Patent Publication No. 03-264530, metal phthalocyanine polysulfonic acid (more precisely, copper phthalocyanine tetrasulfonic acid and nickel phthalocyanine tetrasulfonic acid are exemplified) is disclosed to be effective against retroviruses. Further, Japanese Unexamined Patent Publication No. 2005-9065 discloses that phthalocyanine blue (i.e. copper phthalocyanine) is effective against coronavirus, SARS, herpesvirus, AIDS (Acquired Immunodeficiency Syndrome) virus, adenovirus, HBV (Hepatitis B Virus) and HCV (Hepatitis C Virus).

Japanese Unexamined Patent Publication No. 03-264530 discloses that phthalocyanine tetrasulfonic acid having a divalent metal ion as a central metal has a growth inhibitory effect on a retrovirus. It is thought that the phthalocyanine tetrasulfonic acid inhibits the reverse transcription (RT) activity peculiar to the retrovirus (HIV), and thereby suppressing the growth of the retrovirus. In addition, as antiretroviral agents, agents that include the reverse transcriptase (RT) inhibitors or protease inhibitors are commonly used. However, there has been no report on a growth inhibitory effect on a virus that grows, like influenza viruses, with the help of the activity of the HA (hemagglutinin) and NA (neuraminidase) spikes on the surface of the virus particle.

On the other hand, Japanese Unexamined Patent Publication No. 2004-357871 discloses that masks made of cloth carrying metal phthalocyanine derivatives have disinfecting and deodorizing effects.

### Summary of the Invention

The present inventors have long been studying the enzyme-like catalytic activity of metal phthalocyanines and have found that protein denaturation is induced by the adsorptive properties and redox catalytic functions of the metal phthalocyanines. Considering that it is difficult to completely eliminate emergence of influenza viruses, an object of the present invention is to provide antiviral agents effective against viruses that are far smaller in size than that of bacteria by utilizing the properties of the metal phthalocyanines, and to provide antiviral fiber products and fiber structures both of which carry the antiviral agents.

An an tiviral agent effective against an influenza virus developed to solve the above-mentioned problems comprises metal phthalocyanine represented by the following formula 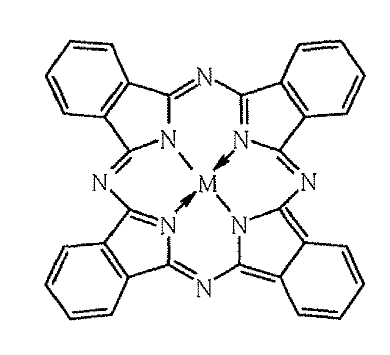 in the formula, M is a metal selected from the group consisting of Fe, Co, Mn, Ti, V, Ni, Cu, Zn, Mo, W and Os.

Another antiviral agent effective against an influenza virus comprises a metal phthalocyanine derivative represented by the following formula I 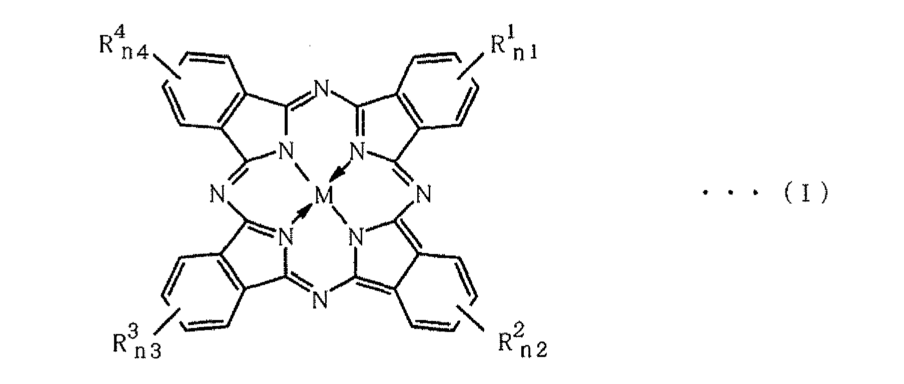 in the formula I, M is a metal selected from the group consisting of Fe, Co, Mn, Ti, V, Ni, Cu, Zn, Mo, W and Os; R¹, R², R³ and R⁴ are the same or different and are each -COOH or -SO₃H group; and n1, n2, n3 and n4 are each 0 to 4 under a condition of 1≤n1+n2+n3+n4≤8 where the sum is a positive number.

The antiviral agents are effective against the influenza virus, and the influenza virus is an avian influenza virus.

The antiviral agent is effective against the avian influenza virus, and the avian influenza virus is at least one kind of virus selected from the group consisting of A/whistling swan/Shimane/499/83 (H5N3) strain and A/Turkey/Wisconsin/1/66 (H9N2) strain.

The antiviral agents are effective against the influenza virus, and the influenza virus is a human influenza virus.

The antiviral agents are effective against the influenza virus, and the influenza virus is a swine influenza virus.

Still another antiviral agent comprises the metal phthalocyanine derivative represented by the above mentioned formula I in which M is a metal selected from the group consisting of Fe, Co, Ni and Cu; R¹, R², R³ and R⁴ are the same or different and are each -COOH or -SO₃H group; and n1, n2, n3 and n4 are each 0 to 4 under a condition of 1≤ n 1+n2+n3+n4≤4 where the sum is a positive number.

An antiviral fiber developed to solve the above-mentioned problems carries an antiviral agent comprising a metal phthalocyanine derivative represented by the following formula I as an effective component 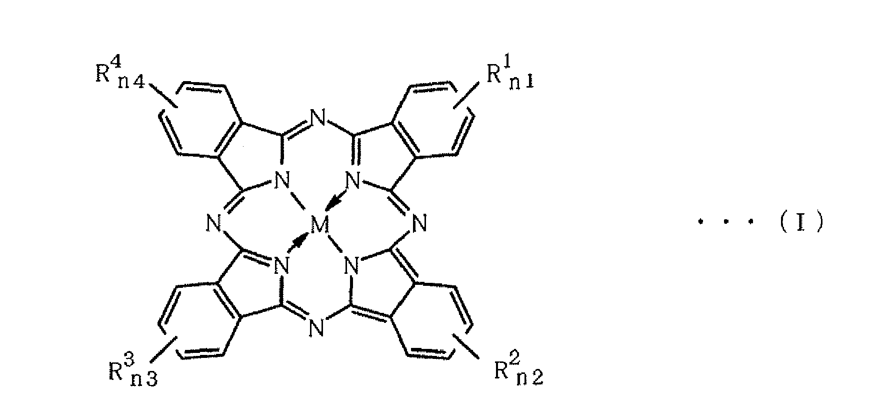 in the formula I, M is a metal selected from the group consisting of Fe, Co, Mn, Ti, V, Ni, Cu, Zn, Mo, W and Os; R¹, R², R³ and R⁴ are the same or different and are each -COOH or -SO₃H group; and n1, n2, n3 and n4 are each 0 to 4 under a condition of 1≤ n1+ n2+ n3+ n4 ≤ 8 where the sum is a positive number.

Likewise, another antiviral fiber carries a metal phthalocyanine derivative represented by the formula I in which M is Fe; R¹, R², R³ and R⁴ are the same or different -COOH group; and n1, n2, n3 and n4 are each 0 to 4 under a condition of 1≤ n1+n2+n3+n4≤ 4 where the sum is a positive number.

Still another antiviral fiber carries the metal phthalocyanine derivative represented by the formula I in which M is Co; R¹, R², R³ and R⁴ are the same or different -SO₃H group; and n1, n2, n3 and n4 are each 0 to 1 under a condition of 1≤ n1+n2+n3+n4≤ 2 where the sum is a positive number.

An antiviral fiber structure of the present invention developed to solve the above-mentioned problems comprises at least partly the antiviral fiber.

Further, an antiviral fiber product of the present invention developed to solve the above-mentioned problems comprises at least partly the antiviral fiber and is formed into clothes, bedclothes, curtains, wallpapers, carpets, mats, sheets, filters, masks, wipers, towels, protective clothes, guard nets, culled chicken bags, poultry house supplies, medical sheets, etc.

The antiviral agent of the present invention is effective in inactivating the influenza viruses. In addition, the fiber carrying the antiviral agent is effective in inactivating the influenza viruses. Further, the antiviral fiber of the present invention can be processed into fiber products and made into the fiber structure of the present invention. The fiber structure has the inactivating effect on the viruses when the viruses are contacted with this fiber structure.

### Detailed Explanation of the Invention

The antiviral agent of the present invention is an agent effective against the influenza viruses and comprises the metal phthalocyanine represented by the following formula 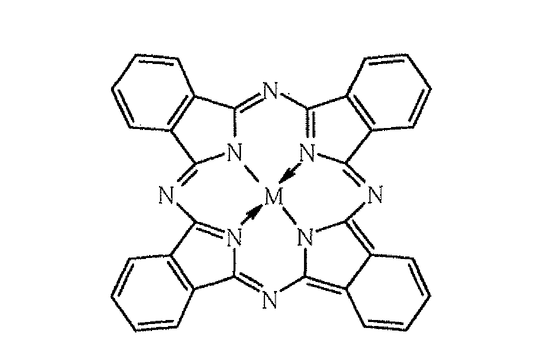 in the formula, M is a metal selected from the group consisting of Fe, Co, Mn, Ti, V, Ni, Cu, Zn, Mo, W and Os, or comprises the metal phthalocyanine derivative represented by the following formula I 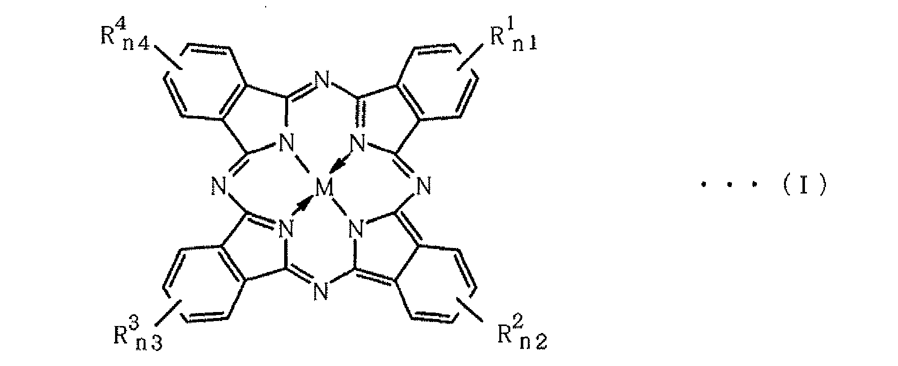 in the formula I, M is a metal selected from the group consisting of Fe, Co, Mn, Ti, V, Ni, Cu, Zn, Mo, W and Os; R¹, R², R³ and R⁴ are the same or different and are each -COOH or -SO₃H group; and n1, n2, n3, and n4 are each 0 to 4 under a condition of 1≤ n1+n2+n3+n4≤ 8 where the sum is a positive number.

The metal phthalocyanine derivatives in the present invention are metal phthalocyanine compounds and their salts having structure mentioned above. As the salts of the metal phthalocyanine compounds, salts of inorganic bases or organic bases etc. can be exemplified. As preferable examples of the salts of inorganic bases, alkali metal salts such as sodium salts, potassium salts, etc.; alkaline earth metal salts such as calcium salts, magnesium salts, etc.; and copper (II) salts; and ammonium salts can be exemplified. As preferable examples of the salts of organic bases, salts of trimethylamine, triethylamine, pyridine, picoline, ethanolamine, diethanolamine, triethanolamine, dicyclohexylamine, etc., can be exemplified.

The antiviral agents are effective against the influenza viruses and are able to inactivate the avian influenza virus. They are especially effective against highly-virulent, highly-pathogenic avian influenza viruses such as H5 and H7 subtype viruses. In the present invention, antiviral effects on the avian influenza viruses of A/whistling swan/shimane/499/83 (H5N3) strain and A/Turkey/Wisconsin/1/66 (H9N2) strain are confirmed. The antiviral agents are thought to be effective against other avian influenza virus such as H5N1 type.

The antiviral agents of the present invention have high inactivating effect on human influenza viruses as well as all sorts of influenza viruses. The antiviral agents of the present invention are proved to have the antiviral effect on the human influenza virus of A/Aichi/2/68 (H3N2) strain and are also thought to be effective against other human influenza viruses.

The antiviral agents of the present invention are proved to be effective against the swine influenza virus of A/Swine/lowa/15/30 (H1N1) strain.

The reason why the antiviral agents of the present invention have an inactivating effect on influenza viruses is considered that the activity of the HA (hemagglutinin) and NA (neuraminidase) spikes on the surface of the influenza virus is inhibited by the agents. In particular, when a functional group such as -COOH or -SO₃H group is introduced into metal phthalocyanine, the inhibiting effect on the HA and NA activities is considered to be increased.

In the formula I, when M is Fe; R¹, R², R³ and R⁴ are all -COOH group, and n1, n2, n3 and n4 are all 1, following formula III is obtained.

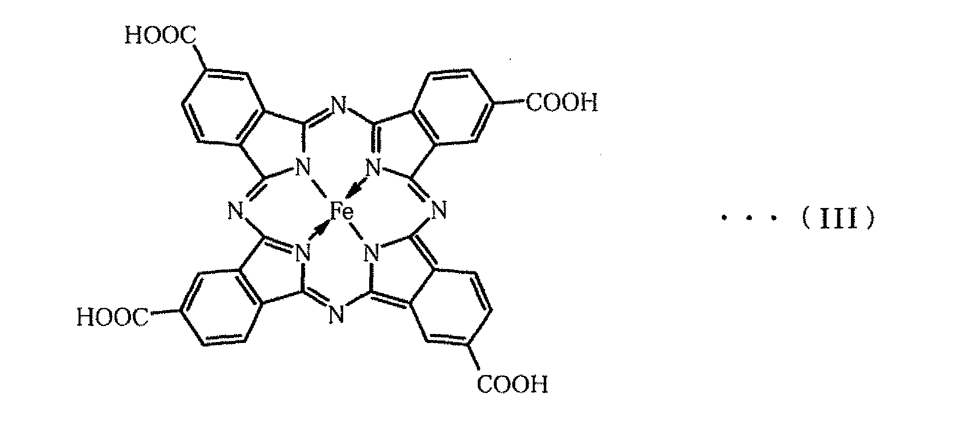

This iron phthalocyanine tetracarboxylic acid can be synthesized as follows. Trimellitic acid anhydride, urea, ammonium molybdate and ferric chloride anhydride are added into nitrobenzene, and the mixture is stirred and refluxed under heating to obtain precipitate. An alkali is added to cause hydrolysis of the resulting precipitate and then acid is added to make it acidic, thereby obtaining the compound.

Likewise, the metal phthalocyanine derivatives represented by the formula I has a structure shown by the following formula IV, when M is Co; and R¹ and R³ are each -COOH group.

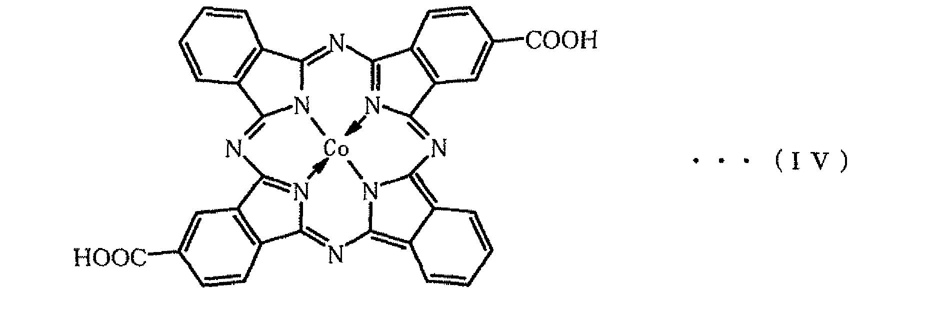

Likewise, the metal phthalocyanine derivatives represented by the formula I has a structure shown by the following formula V, when M is Co; and R¹ and R³ are each -SO₃H group.

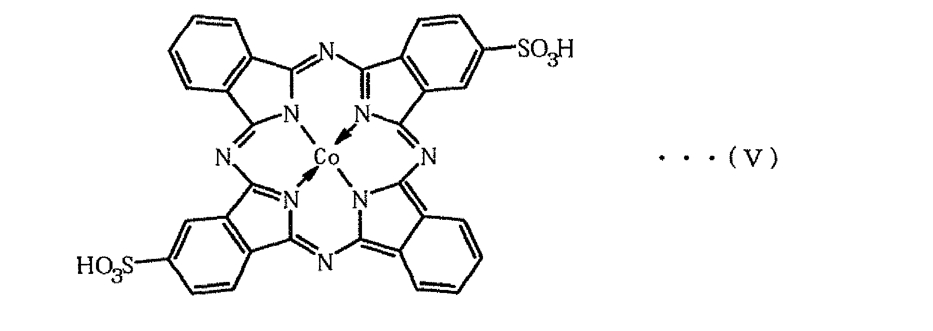

The metal phthalocyanine derivative represented by the formula I has structure shown by the following formula VI, when M is Fe; and R¹ and R³ are each -SO₃H group.

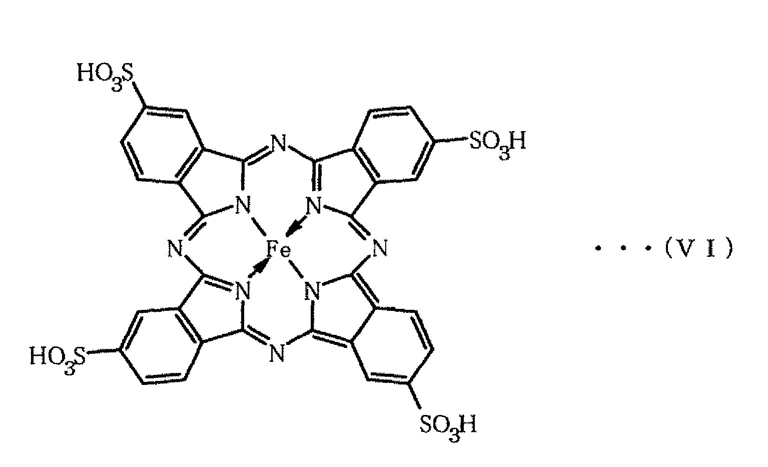

The metal phthalocyanine derivative represented by the formula I has structure shown by the following formula VII, when M is Fe; R¹, R², R³ and R⁴ are all -COON group; and n1, n2, n3 and n4 are each 2.

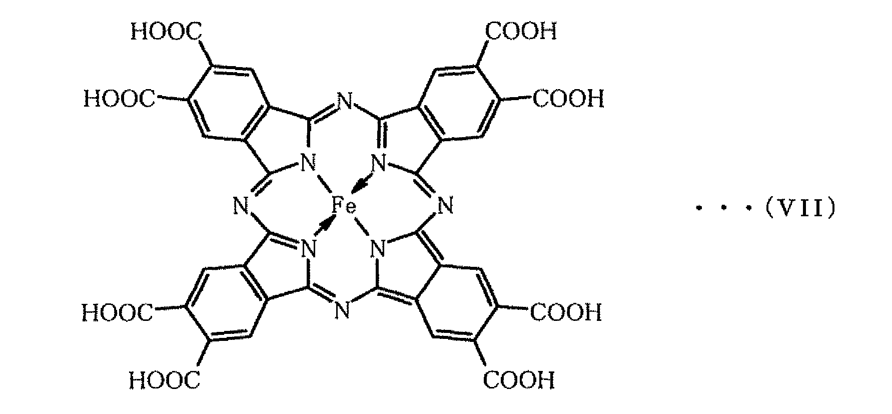

These metal phthalocyanine derivatives, which are used as the antiviral agents, can be manufactured by publicly known technologies and are commercially available as dyestuffs or functional substances having an enzyme-like functions, etc. The derivatives can be manufactured by a method described in, for example, "Phthalocyanine -- Chemistry and Function --" (by Hirofusa Shirai and Nagao Kobayashi, published by Industrial Publishing & Consulting, Inc. (IPC) on February 28, 1997).

For example, iron phthalocyanine tetracarboxylic acid can be manufactured by adding trimellitic acid anhydride, urea, ammonium molybdate and ferric chloride anhydride into nitrobenzene. The mixture is stirred and refluxed under heating to obtain precipitate. An alkali is added to cause hydrolysis of the resulting precipitate, and then acid is added to make it acidic, thereby obtaining the compound.

The cobalt phthalocyanine octacarboxylic acid can be manufactured likewise by using pyromellitic acid anhydride instead of trimellitic acid anhydride, and cobaltic chloride instead of ferric chloride anhydride, which are ingredients of the above mentioned iron phthalocyanine tetracarboxylic acid.

The number of the functional groups or the whole sum of from n1 to n4 of the metal phthalocyanine derivative is preferably 4 or less. More preferably, the number of the functional groups is one or two. When the number of the functional groups is 4 or less, antiviral effect tends to be increased. In particular, when M is Fe, a preferred functional group is -COOH. When M is Co, a preferred functional group is -SO₃H. The metal phthalocyanine derivatives such as iron (III) phthalocyanine tetracarboxylic acid represented by the formula III, cobalt (II) phthalocyanine monosulfonic acid and cobalt (II) phthalocyanine disulfonic acid, have, in particular, the high antiviral effect. The reason why the above mentioned metal phthalocyanine derivatives have the high antiviral effect has not yet clarified, but these differences are considered to be attributed to the special configuration of these metal phthalocyanines.

The metal phthalocyanine derivatives can also be used as the antiviral agent by being carried or blended with an organic or inorganic carrier. The content of the metal phthalocyanine derivative with respect to that of the carrier is not particularly limited as long as it can be carried or blended, and antiviral effect is not impaired. For example, preferably 0.1 to 10% by mass of the metal phthalocyanine derivative with respect to the carrier is used, more preferably 0.3 to 5% by mass, still more preferably 0.5 to 3% by mass. When the metal phthalocyanine derivatives are used within the range mentioned above, the antiviral effect can be fully exerted.

As the organic carrier, fiber is preferably used. The fiber is basically bulky and has a large surface area so that the metal phthalocyanine or its derivatives can be effectively contacted with the viruses in the air.

As fiber materials, all sorts of natural fiber, regenerated fiber, semisynthetic fiber and synthetic fiber such as cellulosic fiber (cotton, hemp, rayon, pulp etc.), protein fiber, (wool, silk etc.), polyamide fiber, polyester fiber, polyacrylic fiber, polyvinyl alcohol fiber, polyvinyl chloride fiber, polyvinylidene chloride fiber, polyolefin fiber, polyurethane fiber, etc. can be used. Among them, the cellulosic fiber, in particular, cotton or rayon, can be preferably used, because they provide a good environment for the metal phthalocyanine to exert an enzyme-like functions as such fiber has a good water absorbing property.

The antiviral fiber of the present invention has the inactivating effect on a variety of viruses that are smaller in size than that of bacteria because the antiviral agent is carried with the fiber.

In particular, the antiviral fiber of the present invention has the effect against influenza viruses and can inactivate avian influenza viruses. The antiviral fiber of the present invention, in particular, has an effect against high virulent, high-pathogenic avian influenza viruses such as H5 and H7 subtypes.

The fiber carrying the antiviral agent may consists of an antiviral fiber carrying the antiviral agent comprising the metal phthalocyanine derivative on raw fibers such as the natural fiber, the synthetic fiber, the semi-synthetic and the regenerated fiber. On the other hand, the fiber structure may consists of an antiviral fiber structure carrying the antiviral agent comprising the metal phthalocyanine derivative on the raw fiber structure such as yarn, woven/knitted fabric, webs, nonwoven, paper, nets, etc. which are previously formed from the natural fiber, the synthetic fiber, the semi-synthetic fiber and the regenerated fiber.

As a method of carrying the metal phthalocyanine derivative after fiber structure is formed, a process such as printing, spraying or coating a solution comprising the metal phthalocyanine derivative; dipping the fiber structure into the solution; or dyeing such as direct dyeing or ionic dyeing; can be adopted. The ionic dyeing is a process in which cationic groups are bonded to fiber such as cotton, rayon etc. and then the cationic groups are ionically connected to anionic groups such as the carboxyl group or the sulfone group of thus dyestuff.

With regard to the fiber material carrying the metal phthalocyanine derivative, it is preferable that the raw fiber material be previously subjected to a cationization treatment. The cationization treatment improves the carrier effect of the metal phthalocyanine derivative and further enhances the antiviral effect because the metal phthalocyanine derivative is held at a high active state.

As for a cationizing agent used for the cationization treatment, for example, quaternary ammonium salt type chlorohydrin derivatives, quaternary ammonium salt type polymers, cationic polymers, cross-linked type polyalkylimines, polyamine type cation resins, glyoxal fibrin reaction type resins, etc. can be exemplified. These cationizing agents can be used alone or in combination of two or more. Preferred cationizing agents are the quaternary ammonium salt type chlorohydrin derivatives.

The antiviral fiber can be used for example by adding the fiber into sheet-like articles, molded resin articles, molded inorganic articles etc. In addition, the fiber can be laminated to, for example, sheet-like articles, molded resin articles, molded inorganic articles using binders, etc.

The antiviral fiber can be used in any cross-sectional shape such as a circular-shape, irregular-shape, or hollow-shape which, however, is not limitative. And their fiber length is not specifically limited, that is, any fiber such as long, short or fine fiber can be used. The long fiber can be obtained by winding it on a bobbin as it is after the fiber is spun. On the other hand, the short fiber can be obtained by cutting fiber into a predetermined length using a cutter. When the fiber is the natural fiber, the fiber can be used as it is. The fine fiber can be obtained by grinding the fiber using a grind mill and then the ground fiber is classified using a screen having an appropriate mesh size. The ground and cut fine fiber has a moderate curvature. In addition, the fineness of the antiviral fiber is not specifically limited, and the fiber having any fineness range can be used in accordance with an intended use.

The antiviral fiber structure of the present invention contains at least partly the antiviral fiber therein and can be used by forming it into yarn, woven/knitted fabric, webs, nonwoven, paper, nets, etc. In addition, the fiber structure may be laminated with another sheet such as a film etc. to form a laminated sheet.

The antiviral fiber is included at least partly within, for example, fiber products such as clothes (including hats, gloves, handkerchiefs), bedclothes (including futon/Japanese style bedding, pillows), curtains, wallpapers, carpets, mats, sheets, filters, masks, wipers, towels, protective clothes, guard nets, culled chicken bags, poultry house supplies. These fiber products are offered for our daily lives and are used to inactivate the viruses that are scattering and floating in our living spaces.

The antiviral fiber structure and antiviral fiber product of the present invention will be explained precisely. In a case where the antiviral fiber structure of the present invention is made from the nonwoven, the fiber webs thereof can be formed, for example, by a process of carding, airlaiding, wet-paper forming, spunbonding, meltblowning, flash spinning, or electrospinning. The resulting fiber webs are processed into an airthrough nonwoven, a thermobonded nonwoven such as a thermally pressure-bonded nonwoven, a chemical bonded nonwoven, a needlepunched nonwoven, a hydroentangled nonwoven, a spunbonded nonwoven, a meltblown nonwoven, etc.

In a case where the antiviral fiber for the fiber web previously carrying the antiviral agent is used, the fiber web thereof may be formed from 100% by mass of the antiviral fiber, but can be formed from a mixture of the antiviral fiber of the present invention and another antiviral fiber or can be mixed with another fiber, as long as the antiviral effect is exerted. When the antiviral fiber is mixed with another fiber, the amount of the antiviral fiber is preferably included in an amount of at least 20% by mass, more preferably at least 30% by mass, still more preferably 50% by mass or more. The thus obtained fiber webs can be subjected to a predetermined process of being formed into the nonwoven.

For example, in a case where fiber as a carrier (hereinafter it is also called "fiber as carrier"), a fiber web and raw nonwoven are prepared thereby obtaining a nonwoven carrying the antiviral agent, the fiber web may consist of 100% by mass of the fiber as carrier. However, the fiber as carrier may be mixed with another antiviral fiber or another fiber within a range where antiviral effect is exerted. When mixed with another fiber, at least 20% by mass of the fiber as carrier is preferably used, more preferably at least 30% by mass, still more preferably 50% by mass or more. Like this, in a case where the fiber web or nonwoven is prepared to carry the antiviral agent in a post processing step, unification of the nonwoven with another sheet through lamination preferably improves the physical strength and processability of the nonwoven, and accordingly production rate can be improved. As an examples of the another sheet, a spunbonded nonwoven, meltblown nonwoven, drawn uniaxially arranged nonwoven whose filaments are all arranged in one direction, cross-laminated nonwoven whose filaments are laminated in such a manner that directions of the filaments are disposed perpendicular to each other, paper prepared by a paper-making technique, nets, film, woven/knitted fabric, can be exemplified. As a preferred reinforcing layer, the spunbond nonwoven, the drawn uniaxially arranged nonwoven and the cross-laminated nonwoven are specifically exemplified because they give physical strength to the laminated nonwoven.

The antiviral fiber structures of the present invention may include another antiviral fiber. As another fiber carrying a substance having an antiviral effect, a fiber having, for example, a carboxyl group within its molecule, more specifically, a fiber having as a component an antiviral substance comprising a polymer containing a maleic acid component as a monomer unit in the polymer chain, can be exemplified. As a preferred polymer in the another antiviral fiber (hereinafter also called as "another antiviral fiber M"), an olefine-maleic acid copolymer, a styrene-maleic acid copolymer, a vinylester-maleic acid copolymer, a vinyl acetate-maleic acid copolymer and a vinyl chloride-maleic acid copolymer are exemplified. Further, these polymers preferably carry an ion of a metal selected from the group consisting of copper, silver and zinc.

As the fiber as carrier for the another antiviral fiber M, all sorts of natural fiber, regenerated fiber, semisynthetic fiber and synthetic fiber, such as cellulosic fiber (cotton, hemp, rayon, pulp, etc.), proteinic fiber (wool, silk etc.), polyamide fiber, polyester fiber, polyacrylic fiber, polyvinyl alcoholic fiber, polyvinyl chloride fiber, polyvinylidene chloride fiber, polyolefin fiber, polyurethane fiber can be used. Among them, the cellulosic fiber is preferably used because the cellulosic fiber has excellent properties mentioned above. Further, the cellulosic fiber does not collect dust by electrostatic occurrence like synthetic fiber. Therefore, as for the cellulosic fiber, a reaction site cannot be blocked by dust, being able to fully exert the antiviral action. The rayon, in particular, has an excellent water-absorbing property and is easy to adjust its fineness and length so that rayon fiber can be used for various fiber structures and fiber products.

A solution of the vinyl acetate-maleic acid copolymer is mixed with and solved into a metal-containing alkaline solution such as a viscose solution or a cuprammonium solution of the cellulose. Both solutions can be prepared by publicly known techniques. Then the resulting liquid mixture is extruded into a spinning solution through a spinning nozzle to obtain the antiviral fiber M by the so-called wet spinning method (for example, Japanese Unexamined Patent Publication No. 08-13905).

A blend ratio of which mass of the cellulose is 60-99% by mass and mass of the copolymer is 40-1% by mass is preferable. When the ratio of cellulose is less than 60% by mass, the surface of the resulting cellulosic composition may become sticky to likely cause disadvantages such as blocking phenomena during subsequent steps such as spinning, weaving and composite-material preparing processes. On the other hand when the ratio of the cellulose exceeds 99% by mass, the antiviral effect induced by the vinyl acetate-maleic acid copolymer may be lowered.

In order to further improve the antiviral function, another antiviral fiber M is preferably carrying at least one kind of a metal ion selected from the group consisting of copper ion, silver ion and zinc ion, by dipping or coating with a solution containing an ion of metal selected from the group consisting of copper, silver and zinc. As the metal ion, the copper ion is preferable due to its excellent antiviral effect. The antiviral fiber M carrying the copper ion can be prepared by immersing it into a solution containing, for example, copper sulfate (CuSO₄) or copper nitrate (Cu(NO₃)₂) to make the fiber M absorb the copper ion. The antiviral fiber M carrying the zinc ion can be prepared by immersing the fiber M into zinc chloride (ZnCl₂) solution.

In a case where the antiviral fiber is used together with above mentioned another antiviral fiber M, the antiviral fiber structure of the present invention can be prepared by a step of previously blending the antiviral fiber of the present invention with the another antiviral fiber M; or in addition to this step, a step of which the another antiviral fiber M is contacted with a metal ion solution to carry the metal ion on the another antiviral fiber M; or a step of which respective fibers as carrier are firstly prepared and then the maleic acid component-containing polymer and the phthalocyanine derivative are applied respectively.

As an example of the antiviral fiber structure of the present invention, the thermobonded nonwoven will be shown. The thermally-bond nonwoven can be manufactured by firstly forming a fiber web by blending the antiviral fiber of the present invention, a thermal adhesive fiber and if necessary another antiviral fiber and/or another fiber; then the thermal adhesive fiber is thermally adhered by heat to obtain the thermobonded nonwoven. As the thermally adhesive fiber, a single component fiber or a composite fiber made from polymers or copolymers comprising, for example, polyester such as polyethylene terephthalate, polybutylene terephthalate, polytrimethylene terephthalate, polylactic acid etc.; polyamide such as nylon 6, nylon 66 etc.; and polyolefin such as polypropylene, polyethylene, polybutene etc., are exemplified. Such polymers or copolymers are at least partly exposed at the surface of the fiber.

A chemical bonded nonwoven will be shown below as an example of the antiviral fiber structure of the present invention. Firstly, a fiber web is formed by mixing the antiviral fiber of the present invention and, if necessary, another antiviral fiber and/or another fiber; then if necessary the fiber web is formed into nonwoven (such as, needlepunched nonwoven); and then a binder is applied by immersing, spraying (for example spray bonding), coating (for example, foam bonding) etc. and then drying and/or curing step is carried out, thereby obtaining the chemical bonded nonwoven. As the binder, an acrylic binder, an urethane binder etc. can be used. The coating weight of the binder is not specifically limited as long as the nonwoven maintains its configuration, and the binder does not impair the antiviral effect. For example, a preferred solid content of the binder with respect to the mass of the nonwoven is in the range of 5-50% by mass.

As still another example of the antiviral fiber structure of the present invention, the hydro-entangled nonwoven will be shown below. Firstly a fiber web is formed by mixing the antiviral fiber of the present invention and if necessary another antiviral fiber and/or another fiber. To the fiber web, another sheet can be laminated, if necessary. As the another sheet, for example, a spunbonded nonwoven, a meltblown nonwoven, a drawn uniaxially arranged nonwoven, a cross-laminated nonwoven, paper prepared by a wet paper-making technique, nets, films and a woven/knitted fabric can be exemplified. The spun-bond nonwoven, the drawn uniaxially-arranged nonwoven and the cross-laminated nonwoven are specifically preferable as a reinforcing layer because they give physical strength to the laminated nonwoven. The fibers can be entangled, for example, by jetting a water stream with a water pressure of 1MPa-10MPa on both front and back surfaces of the fiber web or the laminated sheet one to four times using a nozzle with orifices having pore diameter of 0.05-0.5mm that are arranged at an interval of 0.5mm-1.5mm distance from each other.

In the antiviral fiber structure of the present invention, when a waterproof property is needed, a waterproof antiviral fiber structure can be manufactured by laminating a waterproof film or resin to the surface of the antiviral fiber structure using, for example, an extrusion laminator. Further, when a moisture permeable waterproof property is needed, another antiviral fiber structure having the moisture permeable waterproof property can be obtained by laminating an ultra fine fiber nonwoven such as the meltblown nonwoven or by laminating a moisture permeable resin.

The antiviral fiber structures having a waterproof or moisture permeable waterproof property can be used for medical fabrics such as bed sheets, curtains at hospitals, surgical sheets, experimental sheets, etc.

The thermobonded nonwoven, the chemical bonded nonwoven, the hydro-entangled nonwoven mentioned above can be used as an air filter. When used as the air filter for medium sized dust, preferred fineness of the fiber is in the range of 2-50dtex. Preferred mass per unit area is in the range of 10-150g/m². These air filters obtained above can be used as home appliance filters such as for air conditioners, air purification systems, vacuum cleaners. For example, when used as the filter for the air purification system, the filter has a laminated structure comprising a carrier member such as a chemical bonded nonwoven, etc., the antiviral fiber structure, a high performance filter layer such as an electret nonwoven, a High Efficiency Particulate Air filter (HEPA) or Ultra Low-Penetration Air (ULPA) etc. These are used as pleats-shaped sheets.

The antiviral fiber structure of the present invention can also be used as masks such as sanitary masks, surgical masks, dust masks (for example, corresponding to respiratory protective device (Particulate Respirator Type N95)). As the antiviral fiber structure that can be used for the masks may be made of the aforementioned thermobonded nonwoven and the hydro-entangled nonwoven. The antiviral fiber used for the mask preferably has fineness of 1-10dtex, more preferably 2-8dtex. Preferred mass per unit area is in the range of 30-60g/m². When the mask has a laminated structure comprising, for example, from outside to mouth side, a reinforcing nonwoven (for example, a spunbonded nonwoven, a thermobonded nonwoven)/ the antiviral nonwoven of the present invention/ an ultra fine fiber nonwoven (for example a meltblown nonwoven)/ a reinforced or flexible nonwoven (for example, a spunbonded nonwoven of a thermobonded nonwoven), the antiviral performance can be effectively exerted.

As another example of the antiviral fiber structure of the present invention, a nonwoven made of another fiber, on the surface of which a severed short fiber or a ground fine fiber (hereinafter it is also called as a "fiber powder" generally) made by cutting the antiviral fiber into a fiber having a length less than 5mm, can be exemplified. Another fiber can carry the above-mentioned fiber powder made from the antiviral fiber by using binder on the surface thereof. A method to carrying the antiviral fiber powder using a binder can be carried out by, for example, preparing a binder solution by adding a predetermined amount of the antiviral fiber powder to a binder (for example, an acrylic binder, an urethane binder), then applying the binder solution to a fiber substrate by dipping, spraying (for example spray bonding), coating (for example, using a knife coater or gravure coater), and then drying and/or curing, thus an antiviral fiber powder-carried nonwoven is obtained. As another process, at first, an aqueous dispersion of the antiviral fiber powder is prepared then the aqueous dispersion is applied by dipping, spraying, coating, etc. on a fiber structure containing a heat adhesive fiber or hydrothermal adhesive fiber and then the resulting fiber structure is heated to adhere the powder to the heat adhesive fiber or the heat and humidity adhesive fiber to obtain the antiviral fiber powder-carried nonwoven.

As the fiber substrate, for example, a nonwoven such as a thermobonded nonwoven, a spunbonded nonwoven, a hydro-entangled nonwoven and a woven/ knitted fabric or textile can be used.

The antiviral fiber powder-carried nonwoven can be used as, for example, protective clothes. At first this nonwoven is cut into a predetermined shape and then the cut nonwoven s are overlapped with each other at the end portions thereof and then they are sealed with heat, ultrasonic wave or high frequency wave or by sewing.

The antiviral fabric of the present invention can be used for, for example, clothes, bedclothes (including futon/Japanese-style bedding, pillows), curtains, carpets, mats, sheets, towels, protective clothing, guard nets, culled chicken bags, poultry house supplies, medical sheet materials.

Hereinafter the antiviral fiber carrying antiviral agent will be precisely described with reference to the following Examples. The scope of the present invention is not limited to these Examples.

### -- Manufacture of antiviral fibers --

### [Example 1]

An antiviral rayon fiber was manufactured by a direct staining method. 30kg of rayon fiber (fineness: 2.2dtex, fiber length: 38mm) was dipped into a 600L of cobalt (II) phthalocyanine disulfonic acid disodium salt aqueous solution of 0.3% on weight fiber (owf), (bath ratio = 1:20) and then agitated for 30min. at temperature between 65°C-100°C to stain the rayon fiber. As a dyeing auxiliary, 30g of sodium sulfate was added for 1 liter of the dye solution. And the stained rayon was washed with water, dewatered and dried, obtaining antiviral fibers carrying cobalt (II) phthalocyanine disulfonic acid disodium salt.

### [Example 2]

An antiviral rayon fiber was manufactured by an ionic dyeing method. Into a 10L liquid mixture of 50g/L Cationon UK aqueous solution, which is available from Ipposha Oil Industries Co., LTD. and is trade name, and of 15g/L sodium hydroxide aqueous solution, 1 kg of rayon fiber as same as in Example 1 was dipped and reacted for 45min. at 85°C under a bath ratio of 1:10. The resulting cationized rayon fiber was well washed with water, and then dipped into a 10L of sodium hydroxide solution (pH=12) mixed with 1 part by mass (1% owf) of cobalt (II) phthalocyanine monosulfonic acid and cobalt (II) phthalocyanine_disulfonic acid for 100 parts by mass of the fiber, and agitated for 30min. at 80°C to stain the rayon fiber. The resulting stained rayon fiber was well washed with water, and dried. Thereby an antiviral fiber carrying cobalt (II) phthalocyanine monosulfonic acid sodium salt and with cobalt (II) phthalocyanine disulfonic acid disodium salt was obtained.

### [Example 3]

The cationized rayon fiber in Example 2 was well washed with water and then dipped into a 10L of sodium hydroxide solution (pH=12) mixed with 1% owf of iron (III) phthalocyanine monosulfonic acid and iron (III) phthalocyanine disulfonic acid, and agitated for 30min. at 80°C to stain the rayon fiber. The resulting stained rayon fiber was well washed with water and then dried thereby an antiviral fiber carrying iron (III) phthalocyanine monosulfonic acid sodium salt and iron (III) phthalocyanine disulfonic acid disodium salt was obtained.

### [Example 4]

The cationized rayon fiber in Example 2 was well washed with water and then dipped into a 10L of sodium hydroxide solution (pH=12) mixed with 1% owf of iron (III) phthalocyanine tetra carboxylic acid and agitated for 30min. at 80°C to stain the rayon fiber. The resulting stained rayon fiber was well washed with water and then dried. Thereby an antiviral fiber carrying iron (III) phthalocyanine tetra carboxylic acid was obtained.

### [Comparative Example 1]

In Comparative Example 1, the untreated rayon fiber in Example 1 was subjected to the same test procedures as described in Example 1 to observe if a virus does grow or not.

### [Comparative Example 2]

In Comparative Example 2, the cationized rayon fiber in Example 2 was subjected to the same test procedures as described in Example 2 to observe if a virus does grow or not.

### -- Performance assessment of antiviral fibers against avian influenza viruses --

As a test virus, the avian influenza virus A/whistling swan/shimane/499/83 (H5N3) strain stored at the Research Center of Avian Influenza Virus of Tottori University was used. This avian influenza virus is also called as the avian influenza virus A/Kohakucho/Shimane/499/83 (H5N3).

Each antiviral fiber obtained in the above-mentioned Examples 2 to 4 was cut into about 1.5cm length, and 0.2g of each was put into a polyethylene bag. Into the respective polyethylene bags, 0.6ml virus solution A, which was obtained by diluting the test virus 100 times (or 1,000 times) with Phosphate Buffer Saline (PBS), was added to infiltrate the virus solution into the antiviral fiber. After letting the bags stood still for 10 min. (or 1min. or 30min.) at 4°C, then the virus solution was sampled and diluted 10 times with PBS using a serial dilution method, and 0.2 ml of it was each inoculated into a chorio allantois cavity of 10-day-old embryonated chicken egg (specific pathogen free: SPF). After two-day cultivation, allantoic fluid B was collected and then determined whether there was virus growth or not using chicken hemagglutination reaction.

Virus titer was calculated using the method of Reed & Muench (1938). Virus titers of respective test samples of antiviral fibers are shown in Table 1.

**Table 1**

| Antiviral Fiber | Virus Strain | Amount of Fiber | Reaction Time | Viral Titer (EID₅₀/0.2ml) | | Virus Reduction (%) |
|---|---|---|---|---|---|---|
| | | | | A Solution | B Fluid | |
| Ex. 2 | H5N3 Strain | 0.2 g | 1 min. | 10 ^{7.25} | 10 ^{4.50} | 99.82 |
| Ex. 2 | H5N3 Strain | 0.2 g | 10 min. | 10 ^{7.25} | 10 ^{2.75} | 99.99 |
| Ex. 2 | H5N3 Strain | 0.2 g | 30 min. | 10^{7.50} | 10 ^{1.50} | 99.999 |
| Ex. 2 | H5N3 Strain | 0.2 g | 10 min. | 10 ^{6.25*} | <10 ^{1.75} | >99.99 |
| Ex. 3 | H5N3 Strain | 0.2 g | 10 min. | 10 ^{6.75} | 10 ^{4.75} | 99.0 |
| Ex. 4 | H5N3 Strain | 0.2 g | 1 min. | 10 ^{7.50} | 10 ^{4.75} | 99.82 |
| Ex. 4 | H5N3 Strain | 0.2 g | 10 min. | 10 ^{7.50} | 10 ^{1.75} | 99.999 |
| Comp. Ex. 1 | H5N3 Strain | 0.2 g | 10 min. | 10 ^{6.75} | >10 ^{5.50} | - |
| Comp. Ex. 2 | H5N3 Strain | 0.2 g | 10 min. | 10 ^{7.00} | 10 ^{5.5} | 96.83 |

| | | | | | | |
|---|---|---|---|---|---|---|
| In Table 1, * mark indicates that virus solution A was diluted to 1,000 times. | | | | | | |

As shown in Table 1, viral titer of the allantoic fluid B in which each antiviral fiber was used as shown in Examples 2-4 considerably decreased when compared with virus solution A. The virus reduction rate showed 99% or more. This means that each antiviral fiber of Examples 2-4 have antiviral effect against the avian influenza virus. In the case of the allantoic fluid B of Comparative Example 2, virus titer decreased when compared with that of the virus solution A but the virus reduction was insufficient. In Examples 2 and 4, in particular, an antiviral effect on the avian influenza virus was considerably enhanced.

### [Example 2-A]

### 1. Manufacture of antiviral nonwoven

100% by mass of antiviral fiber of Example 2 was mixed and then a fiber web was formed using a parallel carding machine and then the fiber web was subjected to a hydro-entangling treatment to obtain an antiviral nonwoven having a mass per unit area of 40g/m².

### 2. Manufacture of antiviral masks

The obtained antiviral nonwoven was laid on the upper surface of a polypropylene spunbonded nonwoven, and further polypropylene meltblown nonwoven and polypropylene spunbonded nonwoven were laminated in this order, and then cut into a square of 15cm by 15cm in side length and made it into pleats of three folds. Then the laminated nonwoven was provided with a string at central portions of both lateral end portions thereof, which was used to hook the mask to the ears. Then four end portions of the laminated sheet ends were heat-sealed to obtain an antiviral mask.

The obtained mask was used for seven days, then the antiviral nonwoven was taken out of the mask and virus titer of the avian influenza virus (H5N3 strain) was measured. Virus titer of the allantoic fluid B (<10^{2.25} (EID₅₀/0.2ml)) considerably decreased when compared with that of the virus solution A (10^{6.75} (EID₅₀/0.2ml)). The virus reduction rate was 99.99%. It was confirmed that the mask of the present invention had a long service life.

### -- Performance assessment of antiviral fibers against human influenza viruses --

As a test virus, human influenza virus A/Aichi/2/68(H3N2) strain was used. In a case of nonwoven, 0.2g of test sample was taken out and cut into a square of 1.5cm by 1.5cm in side length; or in a case of fiber, 0.2g of raw stock comprising about 1.5cm length fiber; was prepared. Each sample was put into a polyethylene bag. Into the respective polyethylene bags, 0.6ml of virus solution A, which was obtained by diluting the test virus 100 times with Phosphate Buffer Saline (PBS), was added to infiltrate the virus solution into the antiviral fibers. After letting the bags stand still for 10min. (or 1min.) at 4°C, then the virus solution was sampled and then diluted 10 times with PBS and 0.2ml of it was each inoculated into the chorio allantois cavity of 10-day-old embryonated chicken egg (SPF). After two days cultivation, allantoic fluid B was collected and then determined whether there was virus growth or not using chicken hemagglutination reaction. Virus titer was calculated using the method of Reed & Muench (1938).

Virus titers of human influenza virus of Examples 2-4 are shown in Table 2.

**Table 2**

| Antiviral Fiber | Virus Strain | Amount of Fiber | Reaction Time | Viral titer (EID₅₀/0.2ml) | | Virus Reduction (%) |
|---|---|---|---|---|---|---|
| | | | | A Solution | B Fluid | |
| Ex. 2 | H3N2 Strain | 0.2 g | 10 min. | 10 ^{6.25} | 10 ^{3.5} | 99.82 |
| Ex. 3 | H3N2 Strain | 0.2 g | 10 min. | 10 ^{6.25} | 10 ^{4.0} | 99.44 |
| Ex. 4 | H3N2 Strain | 0.2 g | 10 min. | 10 ^{6.25} | 10 ^{2.5} | 99.98 |

As shown in Table 2, virus titer of the allantoic fluid B, in which each antiviral fiber of Examples 2-4 was used, considerably decreased when compared with that of the virus solution A. Each virus reduction rate was more than 99%. This means that each antiviral fiber of Examples 2-4 has antiviral effect on the human influenza virus.

### --Performance assessment of antiviral fiber against other influenza virusVirus titer of antiviral fiber in Example 4 was observed using the same evaluation method described above except that following viruses were used.

(1) Avian influenza virus A/ Turkey/ Wisconsin/1/66 (H9N2)) strain
(2) Swine influenza virus A/Swine/lowa/15/30(H1N1) strain

**Table 3**

| Antiviral Fiber | Virus Strain | Amount of Fiber | Reaction Time | Viral Titer (EID₅₀/0.2ml) | | Virus Reduction (%) |
|---|---|---|---|---|---|---|
| | | | | A Solution | B Fluid | |
| Ex. 4 | H9N2 Strain | 0.2 g | 10 min. | 10 ^{6,25} | 10 ^{2.50} | 99.98 |
| Ex. 4 | H1N1 Strain | 0.2 g | 10 min. | 10 ^{6.50} | 10 ^{3.50} | 99.9 |

As shown in Table 3, virus titer of allantoic fluid B in which the antiviral fiber of Example 4 was used, considerably decreased when compared with that of the virus solution A. The virus reduction rate was more than 99%. This indicates that the antiviral fiber in Example 4 has antiviral effect on another type of influenza virus.

### [Example 5]

### 1. Manufacture of antiviral nonwoven

Antiviral fiber of Example 4 and core-sheath type composite fiber (fineness: 2.2dtex, fiber length: 51mm), which is available from Daiwabo polytec Co. LTD. and is NBF(H) :trade name, comprising polypropylene as a core material and high-density polyethylene as a sheath material, were used. 60% by mass of antiviral fiber of the present invention and 40% by mass of the core-sheath type composite fiber were mixed and spread using a parallel carding machine. Thus card web was prepared. The mass per unit area of the card web was 40g/m².

Fiber on a surface of the obtained card web was jetted twice with columnar water flow having a water pressure of 4MPa using nozzles with a diameter of 0.08mm and orifices arranged at a spaced interval of 0.6mm from each other and then fiber on the other surface of the card web was also jetted twice with columnar water flow having the same water pressure of 4MPa to cause fiber to be entangled, and then dehydrated using a box type vacuum aspirator. Then the sheath portion of the core sheath type composite fibers was fusion bonded in a drum-type drier at 140 °C of controlled temperature and dried to provide a hydroentangled nonwoven (antiviral nonwoven) which was made into one body by hydro-entanglement.

### 2. Performance assessment of antiviral nonwoven against avian influenza virus

The antiviral nonwoven was taken out and then virus titer was measured. Virus titer of the allantoic fluid B (<10^{2.25} (EID₅₀/0.2ml)) considerably decreased when compared with that of the virus liquid A (10^{6.75} (EID₅₀)/0.2ml)). The virus reduction rate was 99.99%.

### [Example 6]

### 1. Manufacture of water proof antiviral nonwoven

On the antiviral nonwoven of Example 5, a low density polyethylene having a melting point of 103°C was laminated using an extrusion laminator. Thus a water proof antiviral nonwoven having a thickness of 30µm was prepared.

### 2. Manufacture of medical sheets and experimental sheets

The waterproof nonwoven was used as a medical sheet and experimental sheet. Waterproof property and antiviral effects were confirmed.

### [Example 7]

### 1. Manufacture of nonwoven carrying antiviral fiber powder

### (1) Manufacture of antiviral fiber powder

At first, antiviral rayon fiber was obtained as same as in Example 2, then the fiber was cut into 0.1mm length using a cutter to obtain an antiviral fiber powder.

### (2) Manufacture of another antiviral fiber powder

Next, another antiviral fiber M was manufactured according to the following process. Into a viscose solution of cellulose (cellulose concentration is 9%), water-soluble salts of vinyl acetate-maleic anhydride copolymer was added and solved so that the content of the copolymer became 20 parts by mass for 100 parts by mass of cellulose as a solid content. The mixed solutions were extruded using a nozzle made of platinum into a strongly acidic spinning bath comprising 130g/L of sulfuric acid, 10g/L zinc sulfate and 250g/L of sodium sulfate. After conventional desulfurizing, refining and bleaching processes, viscose rayon fiber containing vinyl acetate-maleic acid anhydride copolymer was obtained. The fiber was cut into 0.1mm length using a cutter, thus obtaining an antiviral fiber powder. Its fineness was 3.3dtex.

The obtained fiber powder was immersed into 4% copper sulfate aqueous solution for 10min., washed with distilled water and then dried for 3 hours at 70°C. Thus the powder of antiviral fiber M carrying copper ion was obtained. The powder of the antiviral fiber M had copper content of 1% by mass as a sample.

Virus titer of the obtained antiviral fiber M powder against avian influenza virus (H5N3 strain) was measured. The viral titer of allantoic fluid B (<10^{0.5} (EID₅₀/0.2ml)) considerably decreased when compared with that of virus solution A (10^{7.5}(EID₅₀/0.2ml)). Virus reduction rate was 99.999%.

### (1) Manufacture of nonwoven

As a fiber substrate, polypropylene spunbonded nonwoven having a mass per unit area of 40g/m² was prepared. Next, an acryl emulsion binder solution for adhering onto the fiber substrate was prepared by adding 50% by mass of the powder of the antiviral fiber of the present invention and 50% by mass of powder of other antiviral rayon fiber M into the acryl emulsion binder. On the surface of the fiber substrate, the fiber powder at the rate of 3g/m² was applied using a knife coater. The binder was coated at a rate of 6g/m² in terms of solid content, and then it was cured for 3min. at 150°C. Thus antiviral fiber-carried nonwoven was obtained. Viral titer of the sample of Example 7 of the avian influenza virus is shown in Table 4.

**Table 4**

| Antiviral Fiber | Virus Strain | Amount of Fiber | Reaction Time | Viral Titer (EID₅₀/0.2ml) | | Virus Reduction (%) |
|---|---|---|---|---|---|---|
| | | | | A Solution | B Fluid | |
| Ex. 7 | H5N3 Strain | 0.2 g | 10 min. | 10 ^{6.5} | <10 ^{1.25} | >99.999 |

As shown in Table 4, viral titer of the allantoic fluid B of the sample in Example 7 considerably decreased when compared with that of virus solution A. The virus reduction rate was more than 99%. The results showed that the antiviral fiber powder-carried nonwoven had an antiviral effect against the avian influenza virus.

### 1. Manufacture of protective clothes

The antiviral fiber powder-carried nonwoven of Example 7 was cut into a predetermined shape and then the obtained nonwoven were overlapped with each other at their end portions thereof and then heat-welded, to manufacture the protective clothes. The nonwoven themselves were flexible and there was no drop of the fiber powder, being confirmed that it can be used as a protective clothes.

### [Example 8]

### 1. Manufacture of antiviral nonwoven

### (1) Preparation of antiviral fibers

Antiviral rayon fiber with fineness of 15dex and a length of 64mm and carries cobalt (II) phthalocyanine monosulfonic acid sodium salt and cobalt (II) phthalocyanine disulfonic acid disodium salt was prepared in a similar manner to Example 2.

### (2) Preparation of antiviral fiber M

Test sample of fiber having fineness of 7.8dtex and a fiber length of 51 mm and comprising a viscose rayon blended with vinyl acetate-maleic anhydride copolymer was prepared by spinning in a similar manner to Example 7.

### (3) Manufacture of nonwoven

At first, polyester fiber having fineness of 30dtex and a fiber length of 64mm was prepared and then 40% by mass of the antiviral rayon fiber of the present invention, 20% by mass of antiviral fiber M and 40% by mass of polyester fiber were mixed, spread and made into a card web using a carding machine then a laminated web was prepared by means of cross layer. Next, acrylic binder was sprayed on both surfaces of the laminated web and dried for 1min. at 120°C, and cured for 3min. at 150°C. Thereby chemical bonded nonwoven was obtained. The acryl binder was adhered in an amount of 15% by mass in terms of solid content. The mass per unit area was 60g/m².

### 1. Manufacture of air filters

The obtained chemical bonded nonwoven was cut into a predetermined size and then fixed to a plastic unit, thus a prefilter for an air purifier was obtained.

### [Example 9]

### 1. Manufacture of antiviral nonwoven

### (1) Manufacture of antiviral fibers

The cationized rayon fiber obtained in Example 2 was well washed with water, and dipped into a 10L of sodium hydroxide solution (pH=12) mixed with 1% owf of iron (III) phthalocyanine monosulfonic acid and iron (III) phthalocyanine disulfonic acid, and agitated for 30min. at 80°C to stain the rayon fiber. The resulting stained rayon fiber was well washed with water and then dried, thus obtaining an antiviral fiber having fineness of 5.5dtex and a fiber length of 51 mm which carries iron (III) phthalocyanine monosulfonic acid sodium salt and iron (III) phthalocyanine disulfonic acid disodium salt.

### (2) Preparation of antiviral fiber M

A sample of fiber having fineness of 7.8dtex and a fiber length of 51 mm and comprising viscose rayon blended with vinyl acetate-maleic anhydride copolymer was prepared by spinning in a similar manner to Example 7.

### (3) Manufacture of nonwoven

Further, core-sheath type composite fiber in Example 5 was prepared. 40% by mass of antiviral fiber, 30% by mass of antiviral fiber M and 30% by mass of core-sheath type composite fiber were mixed, spread and made into a card web using a carding machine then a laminated web was prepared by means of a cross layer. And then the laminated web was heat-treated at 140°C using hot air-through processing machine to melt the sheath portion of the core-sheath type composite fiber, thus obtaining a thermobonded nonwoven. The mass per unit area was 60g/m².

Viral titer of the sample of Example 9 against avian influenza virus (H5N3 strain) is shown in Table 5.

**Table 5**

| Antiviral Fiber | Virus Strain | Amount of Fiber | Reaction Time | Viral Titer (EID₅₀/0.2ml) | | Virus Reduction (%) |
|---|---|---|---|---|---|---|
| | | | | A Solution | B Fluid | |
| Ex. 9 | H5N3 Strain | 0.2 g | 10 min. | 10 ^{6.75} | <10 ^{1.5} | >99.999 |

As shown in Table 5, viral titer of allantoic fluid B in which the test sample of Example 9 was used, a considerable decrease when compared with that of the virus liquid A was observed. The virus reduction rate was more than 99%. These results showed that the antiviral nonwoven of Example 9 had the antiviral effect on the avian influenza virus.

### [Example 10]

### 1. Manufacture of antiviral textiles

A cationized rayon fiber of Example 2; a viscose rayon fiber having fineness of 1.4dtex and a fiber length of 38mm obtained by viscose rayon blended with vinyl acetate-maleic anhydride copolymer-and by spinning the mixture in a similar manner to Example 7; and regenerated polyester fiber having fineness of 1.45dex and a fiber length of 35mm, which is available from Teijin Fibers Limited and is Ecopet: trade name, were used. Twenty five (25)% by mass of the cationized rayon fiber, 25% by mass of the rayon fiber containing maleic acid and 50% by mass of the regenerated polyester fiber were mixed to manufacture a single yarn (twist coefficient:4.3-4.5 and yarn number:14). A textile (warp:89 / inch, weft:51 / inch, twill: 3/1) was manufactured using an air jet loom.

The textile mentioned above carries cobalt (II) phthalocyanine monosulfonic acid sodium salt and cobalt (II) phthalocyanine disulfonic acid disodium salt using an ionic dyeing method. And then metal-ion treatment was carried out using a jigger dyeing machine. 0.5% owf of copper sulfate aqueous solution with respect to a mass of the fiber was added and the textile was dipped using the jigger dyeing machine, squeezed by nip rolls, then washed with water in a washing bath, and then dried at 120°C, thereby obtaining a textile sample containing an antiviral fiber M carrying copper ion.

### 1. Manufacture of working clothes

The textile of Example 10 was cut into a predetermined shape and the end portions of the cut textile were overlapped with each other and sewn to make working clothes. The clothes were easy to wear.

### [Example 11]

### 1. Manufacture of antiviral nonwoven

20% by mass of antiviral fiber having fineness of 1.7dtex and a fiber length of 38mm obtained by a similar method to Example 3; 30% by mass of copper-carried viscose rayon fiber (antiviral fiber M) having fineness of 1.7dtex and a fiber length of 38mm obtained by a copper-carried viscose rayon blended with vinyl acetate-maleic anhydride copolymer and then by spinning the blended solution in a similar manner to Example 7; and 50% by mass of a polyester type thermal adhesive composite fiber, which is available from Unitika LTD. and is Melty:trade name, having fineness of 2.2dtex and a fiber length of 51 mm; were used. These fibers were mixed and the mixture was formed into a fiber web using a parallel carding machine and then subjected to hydro-entangling treatment, thus obtaining an antiviral nonwoven having the mass per unit area of 50g/m².

Viral titer of the antiviral nonwoven was measured. Viral titer of allantoic fluid B (10^{0.75} (EID₅₀/0.2ml)) dramatically decreased when compared with that of virus solution A (10^{6.75} (EID₅₆/0.2ml)). The virus reduction rate was 99.999%.

### 1. Manufacture of antiviral masks

The obtained antiviral nonwoven was put on a top surface of a polypropylene spunbonded nonwoven. Further, a meltblown polypropylene nonwoven and a spunbonded polypropylene nonwoven were laminated in this order from bottom to top. The laminated sheet was cut into a square of 15 cm by 15cm in side length, and then folded into 3 pleats. Then the laminated nonwoven was provided with a string at both lateral end portions thereof, which was used to hook the mask to the ears. Then four end portions of the laminated sheet ends were heat-sealed to obtain an antiviral mask.

This mask comprised a protective nonwoven (spunbonded nonwoven)/ultra filtration nonwoven (meltblown nonwoven)/ antiviral nonwoven/protective nonwoven (spunbonded nonwoven). These nonwoven were laminated in this order from outside to inside (i.e. mouthside). The mask was easy to use and gave us no feeling of suffocation when used.

### [Example 12]

### 1. Manufacture of antiviral nonwoven

30% by mass of a viscose rayon fiber of the present invention having fineness of 7.8dtex and a fiber length of 76mm obtained in Example 7 in which the viscose rayon was blended with vinylacetate-maleic anhydride copolymer; 30% by mass of antiviral fiber having fineness of 5.6dtex and a fiber length of 76mm obtained in a similar manner to Example 3; and 40% by mass of core-sheath type composite fiber, which is available from Daiwabo polytec Co. LTD. and is NBF(H) :trade name, having fineness of 2.2dtex and a fiber length of 51mm in which core component was made of polypropylene and sheath component was made of high-density polyethylene; were used.

These fibers were mixed and then the mixture was spread using a parallel carding machine to obtain a card web. Next, the card web was heat-treated at 140°C using a heat roll processing machine comprising a pair of emboss/flat roll to melt the sheath component and to partly pressure (the rate of emboss area is about 18%) the core-sheath type composite fiber to manufacture two kinds of thermobonded nonwoven (antiviral nonwoven). The mass per unit area of the obtained nonwoven was 15g/m² and 30g/m² respectively.

The antiviral nonwovens were subjected to a measurement of viral titer of avian influenza virus. The viral titer of allantoic fluid B (<10^{1.50} (EID₅₀/0.2ml)) was considerably decreased when compared with that of virus liquid A (10^{6.75} (EID₅₀/0.2ml)). The virus reduction rate was 99.999%.

### 2. Manufacture of air filters

A chemical bond nonwoven which was a mixture of polyester fiber and rayon fiber was used as a fiber substrate. On the upper surface of the fiber substrate, hot melt adhesive was sprayed and the obtained antiviral nonwoven was laminated and integrated. Then the hot melt adhesive was sprayed on the antiviral nonwoven and an electret nonwoven (ultra filter layer) having a mass per unit area of about 150g/m² was laminated and integrated by the hot melt adhesive. The obtained sheet was pleats-folded using a pleat processing machine and fixed to a plastic unit, thus obtaining a filter for an air purifier.

### Industrial Applicability

The antiviral agent of the present invention can be used as a raw material for antiviral fiber of the present invention. In addition, antiviral fiber structure of the present invention containing at least partly the antiviral fiber of the present invention can be formed into yarn, textiles, nonwoven, paper, nets etc. and be used for various textile industry. The antiviral fiber structures inactivate viruses, being able to be utilized for medical use.

The antiviral fiber products, which contain at least partly the antiviral fiber, can be formed into and used for clothes, bedclothes (including futon/Japanese-style bedding, pillows), curtains, wallpaper, carpets, mats, sheets, filters, masks, wipers, towels, protective clothes, guard nets, bags for culled chicken, poultry house supplies, medical sheets, etc.

## Claims

1. An antiviral agent effective against an influenza virus comprising metal phthalocyanine represented by the following formula 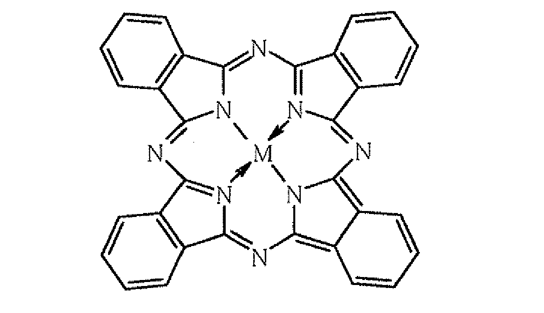 in the formula, M is a metal selected from the group consisting of Fe, Co, Mn, Ti, V, Ni, Cu, Zn, Mo, W and Os.

2. An antiviral agent effective against an influenza virus comprising a metal phthalocyanine derivative represented by the following formula I 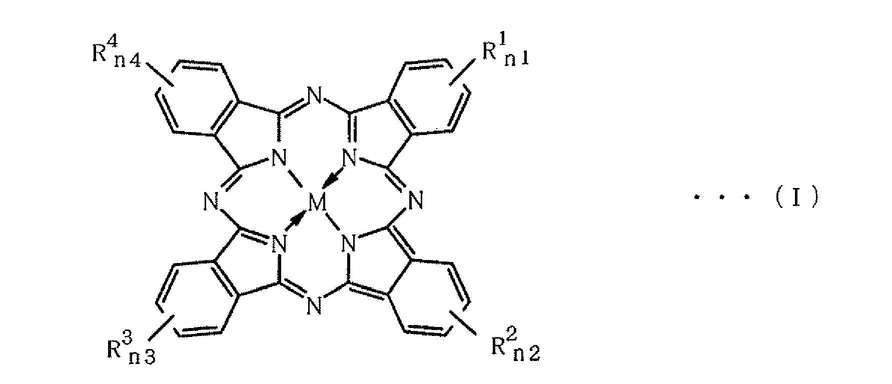 in the formula I, M is a metal selected from the group consisting of Fe, Co, Mn, Ti, V, Ni, Cu, Zn, Mo, W and Os; R¹, R², R³ and R⁴ are the same or different and are each -COOH or -SO₃H group; and n1, n2, n3 and n4 are each 0 to 4 under a condition of 1 ≤ n1 + n2 + n3 +n4 ≤ 8 where the sum is a positive number.

3. The antiviral agent according to claim 1 or 2, wherein the influenza virus is an avian influenza virus.

4. The antiviral agent according to claim 3, wherein the avian influenza virus is at least one kind of virus selected from the group consisting of A/whistling swan/Shimane/499/83 (H5N3) strain and A/Turkey/Wisconsin/1/66 (H9N2) strain.

5. The antiviral agent according to claim1 or 2, wherein the influenza virus is a human influenza virus.

6. The antiviral agent according to claim 1 or 2, wherein the influenza virus is a swine influenza virus.

7. The antiviral agent according to claim 2, wherein in the metal phthalocyanine derivative represented by the formula I, M is a metal selected from the group consisting of Fe, Co, Ni and Cu; R¹, R², R³ and R⁴ are the same or different and are each -COOH or -SO₃H group; and n1, n2, n3, and n4 are each 0 to 4 under a condition of 1≤ n 1 + n2+ n3+ n4 ≤ 4 where the sum is a positive number.

8. An antiviral fiber carrying an antiviral agent comprising a metal phthalocyanine derivative represented by the following formula I as an effective component 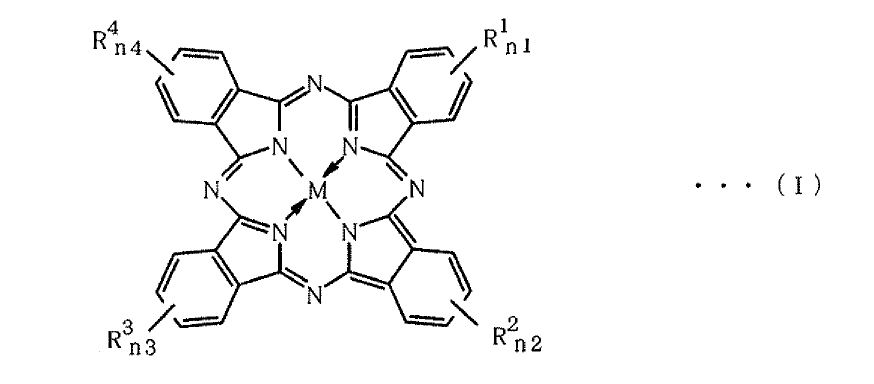 in the formula I, M is a metal selected from the group consisting of Fe, Co, Mn, Ti, V, Ni, Cu, Zn, Mo, W and Os; R¹, R², R³, and R⁴ are the same or different and are each -COOH or -SO₃H group; and n1, n2, n3 and n4 are each 0 to 4 under a condition of 1≤ n1+n2+ n3+ n4≤8 where the sum is a positive number.

9. The antiviral fiber according to claim 8, wherein in the metal phthalocyanine derivative represented by the formula I, M is Fe; R¹, R², R³ and R⁴ are the same or different -COOH group; and n 1, n2, n3 and n4 are each 0 to 4 under a condition of 1≤ n1+n2+n3+n4≤ 4 where the sum is a positive number.

10. The antiviral fiber according to claim 8, wherein in the metal phthalocyanine derivative represented by the formula I, M is Co; R¹, R², R³ and R⁴ are the same or different -SO₃H group; and n1, n2, n3 and n4 are each 0 to 1 under a condition of 1≤ n1+ n2+ n3+ n4≤ 2 where the sum is a positive number.

11. An antiviral fiber structure comprising at least partly the antiviral fiber according to claim 8.

12. An antiviral fiber product comprising at least partly the antiviral fiber according to claim 8 and being formed into clothes, bedclothes, curtains, wallpapers, carpets, mats, sheets, filters, masks, wipers, towels, protective clothes, guard nets, culled chicken bags, poultry house supplies and medical sheets.
